# EUROPEAN PATENT APPLICATION

(11) **EP 1 462 110 A1**
(43) Date of publication of application: **29.09.2004**
(21) Application number: 02782659.3
(22) Date of filing: 04.12.2002
(51) Int. Cl.: A61K 35/78, A61K 35/84, A61P 15/12

(54) **A MEDICINE FOR TREATING WOMAN CLIMACTERIC SYNDROME AND PREPARATION METHOD THEREOF**

(71) Applicant: EU YAN SANG (HONG KONG) LIMITED, Hong Kong (CN)
(72) Inventor: CHAN, Hsiaochang, Hong Kong (CN); GOU, Yulin, Hong Kong (CN); ROWLANDS, Dewi, Hong Kong (CN); CHUNG, Yiuwa, Hong Kong (CN)
(74) Representative: Boff, James Charles
(86) International application number: PCT/CN2002/000868
(87) International publication number: WO 2004/050106

(57) **Abstract**

The present invention discloses a novel medicament for treatment of climacteric syndrome of women, which is prepared from Radix Ginseng, Radix Angelicas Sinensis, Radix Astragali seu Hedysari, Radix Ophiopogonis, Fructus Ligustri Lucidi, Rhizoma Atractylodis Alba, Radix Paeoniae Alba, Poria cocos, Cortex Phellodendri, Rhizoma Ligustici Wallichi, Radix Glycyrrhizae, Radix Bupleuri, Radix Rehmanniae Praeparata, Herba Epimedii, Herba Menthae, Rhizoma Curculiginis, Rhizoma Anemarrhenge, and Herba Ecliptae by processing raw TCM, decocting, drying, grinding, and adding water and honey in a certain proportion to yield aqueous-honey-bolus,

## Description

### Technical Field

The present invention relates to a medicament for treatment of climacteric syndrome of women. More specifically, it relates to a Chinese patent medicine for treatment of climacteric syndrome of women prepared from Chinese herbal medicine as raw materials. The present invention also relates to the preparation method of the medicine.

### Background Art

The climacteric syndrome is used to refer to a series of symptoms and conditions associated with menopause caused by hypofunction of ovary before and after natural menopause, the said symptoms and conditions include malfunction of endocrine system, decrease in estrogen level, disorder of metabolism, menstrual disturbance caused by disturbances of cardiovascular and vegetative nervous system, hidrorrhea, nausea, dizziness and tinnitus, palpitation and insomnia, restlessness and irascibility, soreness of Join and knees, benumbedness and itch of skin, hypomnesis, and even emotional abnormality and the like. It is believed in Traditional Chinese Medicine practitioners that, when women are in climacterium, their bodies gradually transit from a healthy balance towards senility. At the same time, the reduction in kidney-energy, tendency of menstruation consumption, gradual deficit of Chong and Ren channels, insufficiency of vital energy and blood, imbalance of Yin Yang of kidney, which further causes the dysfunction of entrails, cause the above symptoms, which are referred to as "any syndromes before and after menopause". Therefore, kidney is the etiology of this syndrome, which is caused by kidney-asthenia. Kidney comprises kidney-Yin and kidney-Yang, and the abundance of kidney-Yin and kidney-Yang depends on the continuous supplementation of refined substances postnatally derived from spleen and stomach. The regulation of spleen and stomach and the reinforcement of the acquired essential substance to nourish the congenital essential substance are critical for retarding depletion of kidney-energy and eliminating or relieving any syndromes before and after menopause. Therefore, the treatment of the syndrome focuses on nourishing the kidney, with special attention put on the regulation of spleen and stomach, the nourishment of energy and activation of blood, the appropriate relief of liver and stagnation and the regulation of emotions.

A medicament for treatment of climacteric syndrome of women was disclosed in CN 1110164A, which comprised Si Wu Tang (Radix Angelicae Sinensis, Radix Rehmanniae Praeparata, Radix Paeoniae Alba, Rhizoma Ligustici Wallichi), Si Jun Zi Tang (Radix Ginseng, Poria cocos, Radix Glycyrrhizae; Rhizoma AtractyJodis Alba being absent), basic components of Xiao Yao San (Radix Bupleuri, Radix Paeoniae Alba, Radix Angelicae Sinensis, Poria cocos, Radix Glycyrrhizae), Herba Epimedii, and Radix Astragali seu Hedysari. A medicament for treatment of climacteric syndrome of women was disclosed in Xin Zhong Yi, 1988, 20(9), which was consisted of Xiao Yao San and Gan Mai Da Zao Tang and comprised Si Wu Tang (Radix Angelicae Sinensis, Radix Rehmanniae Praeparata, Radix Paeoniae AJba; Rhizoma Ligustici Wallichi being absent), Si Jun Zi Tang (Radix Codonopsis Pilosulae, Poria cocos, Radix Glycyrrhizae, Rhizoma Atractylodis Alba), basic components of Xiao Yao San (Radix Bupleuri, Radix Paeoniae Alba, Radix Angelicae Sinensis, Poria cocos, Rhizoma Atractylodis Alba, Radix Glycyrrhizae), Radix Astragali seu Hedysari and Radix Ophiopogonis. Also, a medicament for treatment of climacteric syndrome of women was disclosed in Xin Zhong Yi, 1988, 26(12), which comprised Herba Epimedii, Radix Ophiopogonis, Fructus Ligustri Lucidi, Rhizoma Atractylodis Alba, Radix Codonopsis Pilosulae, and Rhizoma Curculiginis.

The present invention aims at the climacteric syndrome caused by insufficiency of vital energy and blood, deficit of both kidney-Yin and kidney-Yang, and stagnation of liver energy, which in turn cause menstrual disturbance, occasional intolerance to cold, occasional dizziness and tinnitus, soreness of the waist, hypodynamia, restlessness and irascibility, emotional abnormality, anorexia and fatigue, and feeling of discomfort in chest and hypochondria and the like. The principle of the treatment is to invigorate vital energy, to enrich blood, to invigorate kidney-Yin and kidney-Yang, and to disperse the stagnated liver energy. The anagraph is consisted of Xiao Yao San, Ba Zhen Tang and Er Xian Tang with modification. It can invigorate vital energy and enrich blood, regulate Yin and Yang, disperse the stagnated liver energy, and purge the sthenic kidney-fire. Si Jun Zi Tang (Radix Ginseng, Poria cocos, Radix Glycyrruizae, Rhizoma Atractylodis Alba) and Radix Astragali seu Hedysari are used to invigorate vital energy and strengthen spleen; Si Wu Tang (Radix Angelicae Sinensis, Radix Rehmanniae Praeparata, Radix Paeoniae Alba, Rhizoma Ligustici Wallichi) is used to enrich blood and regulate the menstruation. The association of Si Jun Zi Tang with Si Wu Tang is Ba Zhen Tang, which can invigorate vital energy and enrich blood to treat insufficiency of vital energy and blood. Herba Epimedii and Rhizoma Curculiginis are used to invigorate kidney-Yang; Fructus Ligustri Lucidi and Radix Ophiopogonis are used to invigorate kidney-Yin, the associated use of the four medicaments is able to regulate Yin and Yang. Cortex Phellodendri is used to purge deficiency-fire caused by kidney-Yin deficiency, and Radix Bupleuri and Herba Menthae are used to disperse the stagnated liver energy. The associated use of all of the aforementioned components can result in the invigoration of vital energy and enrichment of blood, balance of Yin and Yang, expedite liver energy, coordination of Chong and Ren channels, thereby any syndromes during menopause can be cured. The present invention is different from CN 1110164A in larger extent, wherein they are different in six components, i.e. Radix Ophiopogonis, Fructus Ligustri Lucidi, Rhizoma Atractylodis Alba, Herba Menthae and Rhizoma Curculiginis.

### Description of Invention

An object of the present invention is to provide a medicament for treatment of climacteric syndrome of women suffered from a series of symptoms associated with menopause that are caused by hypofunction of ovary during menopause, leading to malfunction of endocrine system, decrease in estrogen level, disorder of metabolism, menstrual disturbance caused by disturbances of cardiovascular and vegetative nervous system, hidrorrhea, nausea, dizziness and tinnitus, palpitation and insomnia, restlessness and irascibility, soreness of loin and knees, benumbedness and itch of skin, hypomnesis, and even emotional abnormality and the like.

One aspect of the present invention provides a medicament for treatment. of climacteric syndrome of women, characterized in that it is a pharmaceutical formulation comprising the following raw materials in the following weight proportions:

| | | | |
|---|---|---|---|
| Radix Ginseng | 3.00-3.52 | Radix Angelicas Sinensis | 6.78-7.96 |
| Radix Astragali seu Hedysari | 11.62-13.64 | Radix Ophiopogonis | 4.31-5.05 |
| Fructus Ligustri Lucidi | 4.31-5.05 | Rhizoma Atractylodis Alba | 4.31-5.05 |
| Radix Paeoniae Alba | 5.56-6.52 | Poria cocos | 6.78-7.96 |
| Cortex Phellodendri | 4.31-5.05 | Rhizoma Ligustici Wallichi | 5.56-6.52 |
| Radix Glycyrrhizae | 4.31-5.05 | Radix Bupleuri | 5.56-6.52 |
| Radix Rehmanniae Praeparata | 11.62-13.64 | Herba Epimedii | 6.78-7.96 |
| Herba Menthae | 2.54-2.98 | Rhizoma Curculiginis | 4.64-5.44 |

A second aspect of the present invention provides a medicament for treatment of climacteric syndrome of women, characterized in that it is a pharmaceutical formulation comprising the following materials in the following weight proportions:

| | | | |
|---|---|---|---|
| Radix Ginseng | 3.00-3.52 | Radix Angelicae Sinensis | 6.78-7.96 |
| Radix Astragali seu Hedysari | 9.78-11.48 | Radix Ophiopogonis | 4.31-5.05 |
| Fructus Ligustri Lucidi | 4.31-5.05 | Rhizoma Atractylodis Alba | 3.40-3.98 |
| Radix Paeoniae Alba | 5.56-6.52 | Poria cocos | 4.94-5.80 |
| Cortex Phellodendri | 4.32-5.06 | Rhizoma Ligustici Wallichi | 3.72-4.36 |
| Radix Glycyrrhizae | 3.40-3.99 | Radix Bupleuri | 5.56-6.52 |
| Radix Rehmanniae Praeparata | 11.62-13.64 | Herba Epimedii | 6.78-7.96 |
| Herba Menthae | 2.54-2.98 | Rhizoma Curculiginis | 4.64-5.44 |
| Rhizoma Anemarrhenge | 3.68-4.32 | Herba Ecliptae | 3.68-4.32 |

A further aspect of the present invention further provides a medicament for treatment of climacteric syndroine of women, in which the proportion of materials is as follows, by weight:

| | | | |
|---|---|---|---|
| Radix Ginseng | 3.26 | Radix Angelicae Sinensis | 7.37 |
| Radix Astragali seu Hedysari | 12.63 | Radix Ophiopogonis | 4.69 |
| Fructus Ligustri Lucidi | 4.69 | Rhizoma Atractylodis Alba | 4.69 |
| Radix Paeoniae Alba | 6.04 | Poria cocos | 7.37 |
| Cortex Phellodendri | 4.69 | Rhizoma Ligustici Wallichi | 6.04 |
| Radix Glycyrrhizae | 4.69 | Radix Bupleuri | 6.04 |
| Radix Rehmanniae Praeparata | 12.63 | Herba Epimedii | 7.37 |
| Herba Menthae | 2.76 | Rhizoma Curculiginis | 5.04 |

The medicaments of the present invention may be used in any one of pharmaceutically acceptable oral dosage forms. Specifically, said dosage forms may be tablets, granules, pills, powders, hard gelatin capsules, soft gelatin capsules, elixirs, troches or oral solutions. The present formulations can be prepared by any methods commonly used in the art.

Another object of the present invention is to provide a preparation method of the medicaments for treatment of climacteric syndrome of women. The medicament of the present invention is prepared according to any one of the aforesaid three formulations. Specifically, the method comprises:
1. Radix Ophiopogonis is decocted; Radix Paeoniae Alba is rinsed with water by a high-speed spray gun; Radix Ginseng is ground separatedly; the others are prepared by traditional processes of preparing raw TCM, followed by crushing.
2. Except Radix Ginseng, all materials are mixed together with appropriate amount of adjuvants, such as wine, vinegar, ginger, and salt and the like, followed by decocting in an autoclave.
3. After decocting, the materials are dried at 40-80°C. After drying, they are ground, passed through a sieve of 80 mesh, and mixed with Radix Ginseng powder of the same mesh.
4. The above mixture comprising afore-mentioned powders, are added into water and honey in a certain proportion to prepare aqueous-honey-bolus.

Furthermore, adjuvants may be added to the above mixture in a certain proportion to prepare said oral dosage forms.

The safety and pharmacological activities of medicaments of the present invention are demonstrated by a series of pharmacological experiments.

It is demonstrated by an acute oral toxicity experiment that LD₅₀ of the present medicaments is more than 9000mg/kg, as is the maximum oral dose per day in female Sprague Dawley (SD) rats. This dose corresponds to a dose that is approximately 30-fold higher than the usual oral dose in human. It is demonstrated by a subacute oral toxicity experiment that, during the time period of 28 days, no one death of any rats is observed or no toxic symptom in any rats is manifested when the present medicaments are administered repeatedly at 3000mg/kg to the same line of SD rats.

Significantly elevated level of estrogen is observed in old animals treated with the present medicaments.

The formation of improved folliculus is observed in the ovary of old animals.

The size and weight of pituitaries of old animals re-increased after receiving treatment with the present medicaments. These results suggest that the function of neuro-endocrine system of old animals can be restored by the present medicaments, resulting in re-production of endogenous estrogen, thereby the symptoms associated with impaired production of estrogen in climacterium (i.e. nervous symptoms, osteoporosis) can be relieved or alleviated.

The present medicaments can be hypolipaemic, some ingredients of which are found to have anti-platelet and muscle-relaxing effects. In short, these effects may improve cardiovascular functions.

The present medicaments are shown to restore functions of the immune system in adult animals, suggesting that they may have beneficial effects on the immune system.

The present medicaments and ingredients thereof are found to stimulate the secretion at the colon, indicating that they have an effect on the digestive system.

It has been proved in a preliminary clinical trial that symptoms of climacteric syndrome in women can be improved in a certain extent by daily administration of the present medicaments (300mg/kg p.o.) over a period of 4 to 8 weeks.

### Description of Drawings

Figure 1 is a bar-graph showing effects of the present medicaments on the serum level of estrogen in old SD rats.
Figure 2 is a bar-graph showing effects of the present medicaments on the serum level of progestin in old female SD rats.
Figure 3 is a bar-graph showing effects of the present medicaments on the serum level of β-endorphin in old female SD rats.
Figure 4 is a bar-graph showing effects of the present medicaments on the level of Substance P in old female SD rats.
Figure 5 is a bar-graph showing weight of sexual organs and pituitaries in old female SD rats treated with the present medicaments (3g/kg).
Figure 6 is a photograph showing development of folliculus in the ovary of old female SD rats treated with the medium.
Figure 7 is a photograph showing development of folliculus in the ovary of old female SD rats treated with the present medicaments (3g/kg). The enlarged folliculus are indicated by arrows.
Figure 8 is a bar-graph showing the estrous index of old female SD rats treated with medium two 6-day intervals before and three 6-day intervals after the treatment.
Figure 9 is a bar-graph showing the estrous index of old female SD rats treated with the present medicaments (6g/kg) two 6-day intervals before and three 6-day intervals after the treatment.
Figure 10 is a bar-graph showing effects of the present medicaments on ADP-induced platelet aggregation.

### Special Embodiments

### Example 1

Weight the following raw materials (kg):

| | | | |
|---|---|---|---|
| Radix Ginseng | 3.00 | Radix Angelicae Sinensis | 6.78 |
| Radix Astragali seu Hedysari | 11.62 | Radix Ophiopogonis | 4.31 |
| Fructus Ligustri Lucidi | 4.31 | Rhizoma Atractylodis Alba | 4.31 |
| Radix Paeoniae Alba | 5.56 | Poria cocos | 6.78 |
| Cortex Phellodendri | 4.32 | Rhizoma Ligustici Wallichi | 5.56 |
| Radix Glycyrrhizae | 4.31 | Radix Bupleuri | 5.56 |
| Radix Rehmanniae Praeparata | 11.62 | Herba Epimedii | 6.78 |
| Herba Menthae | 2.54 | Rhizoma Curculiginis | 4.64 |

Firstly, Radix Ophiopogonis is preliminarily decocted then formulated; Radix Paeoniae Alba is rinsed with a high-speed spray gun; Radix Ginseng is ground separatedly and passed through a sieve of 80 mesh; the others are prepared by traditional processes of preparing raw TCM, followed by crushing to the size of 1cm³.

Except Radix Ginseng, all materials are crushed to the size of 1 cm³ and are mixed together with appropriate amount of adjuvants, such as wine, vinegar, ginger, salt and the like, followed by decocting in an autoclave at the temperature of 100°C for 1 hour,

After decocting, the materials are dried evenly in a conveyer belt oven at 80°C, ground in a grinding equipment with air-pipes, passed through a shaking sieve of 80 mesh with three-members vibration, and mixed with Radix Ginseng powder of the same mesh.

The above mixture of the powders is mixed with prefabricated aqueous honey (prepared from honey : pure water 7 : 3) in the ratio of 7 : 3, and prepared aqueous-honey-bolus in a plodder and a bolus-making machine. Then, the bolus are coated and dried at 80°C.

### Example 2

Weight the following raw materials (kg):

| | | | |
|---|---|---|---|
| Radix Ginseng | 3.52 | Radix Angelicae Sinensis | 7.96 |
| Radix Astragali seu Hedysari | 13.64 | Radix Ophiopogonis | 5.05 |
| Fructus Ligustri Lucidi | 5.05 | Rhizoma Atractylodis Alba | 5.05 |
| Radix Paeoniae Alba | 6.52 | Poria cocos | 7.96 |
| Cortex Phellodendri | 5.06 | Rhizoma Ligustici Wallichi | 6.52 |
| Radix Glycyrrhizae | 5.05 | Radix Bupleuri | 6.52 |
| Radix Rehmanniae Praeparata | 13.64 | Herba Epimedii | 7.96 |
| Herba Menthae | 2.98 | Rhizoma Curculiginis | 5.44 |

The preparation method is the same as that in Example 1.

### Example 3

Weight the following raw materials (kg):

| | | | |
|---|---|---|---|
| Radix Ginseng | 3.26 | Radix Angelicae Sinensis | 7.37 |
| Radix Astragali seu Hedysari | 12.63 | Radix Ophiopogonis | 4.69 |
| Fructus Ligustri Lucidi | 4.69 | Rhizoma Atractylodis Alba | 4.69 |
| Radix Paeoniae Alba | 6.04 | Poria cocos | 7.37 |
| Cortex Phellodendri | 4.69 | Rhizoma Ligustici Wallichi | 6.04 |
| Radix Glycyrrhizae | 4.69 | Radix Bupleuri | 6.04 |
| Radix Rehmanniae Praeparata | 12.63 | Herba Epimedii | 7.37 |
| Herba Menthae | 2.76 | Rhizoma Curculiginis | 5.04 |

The preparation method is the same as that in Example 1.

### Example 4

Weight the following raw materials (kg):

| | | | |
|---|---|---|---|
| Radix Ginseng | 3.00 | Radix Angelicae Sinensis | 6.78 |
| Radix Astragali seu Hedysari | 9.78 | Radix Ophiopogonis | 4.31 |
| Fructus Ligustri Lucidi | 4.31 | Rhizoma Atractylodis Alba | 3.40 |
| Radix Paeoniae Alba | 5.56 | Poria cocos | 4.94 |
| Cortex Phellodendri | 4.32 | Rhizoma Ligustici Wallichi | 3.72 |
| Radix Glycyrrhizae | 3.40 | Radix Bupleuri | 5.56 |
| Radix Rehmanniae Praeparata | 11.62 | Herba Epimedii | 6.78 |
| Herba Menthae | 2.54 | Rhizoma Curculiginis | 4.64 |
| Rhizoma Anemarrhenge | 3.68 | Herba Ecliptae | 3.68 |

The preparation method is the same as that in Example 1.

### Example 5

Weight the following materials (kg):

| | | | |
|---|---|---|---|
| Radix Ginseng | 3.52 | Radix Angelicae Sinensis | 7.96 |
| Radix Astragali seu Hedysari | 11.48 | Radix Ophiopogonis | 5.05 |
| Fructus Ligustri Lucidi | 5.05 | Rhizoma Atractylodis Alba | 3.98 |
| Radix Paeoniae Alba | 6.52 | Poria cocos | 5.80 |
| Cortex Phellodendri | 5.06 | Rhizoma Ligustici Wallichi | 4.36 |
| Radix Glycyrrhizae | 3.99 | Radix Bupleuri | 6.52 |
| Radix Rehmanniae Praeparata | 13.64 | Herba Epimedii | 7.96 |
| Herba Menthae | 2.98 | Rhizoma Curculiginis | 5.44 |
| Rhizoma Anemarrhenge | 4.32 | Herba Ecliptae | 4.32 |

The preparation method is the same as that in Example 1.

### Example 6

Effects of the present medicaments on the serum level of hormones in old female SD rats

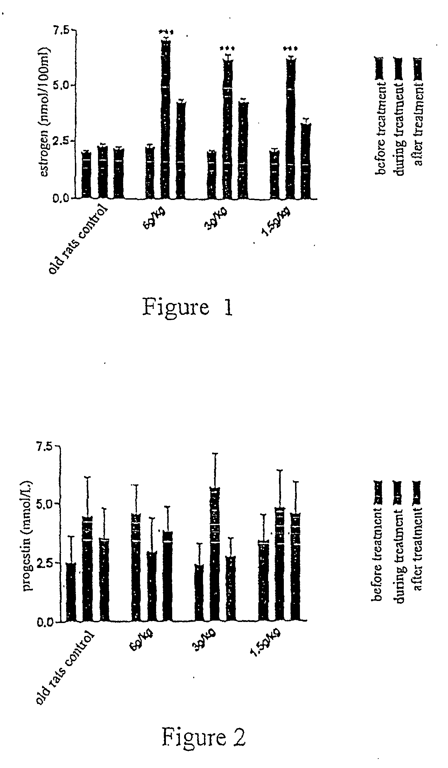

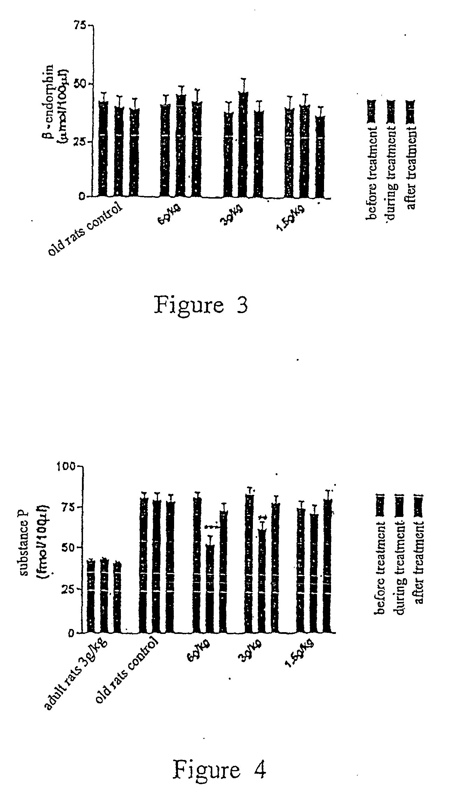

Female SD rats of 18-month age are administrated orally with the present medicaments at 1.5-6g/kg. Blood was collected. Changes in the level of hormones in blood samples are analysed. The result shows that the serum estrogen level can be increased significantly by the present medicaments, while no changes are found in control animals (Fig. 1). The serum level of Substance P is also decreased by oral administration of the present medicaments to SD rats of 18-monlh age at 3g/kg - 6g/kg as compared with control (Fig. 4). Nevertheless, the serum levels of progestin (Fig. 2) and β-endorphin (Fig. 3) remain unchanged at all dose administered.

### Example 7

### Effects of the present medicaments on the reproductive system in old female SD rats

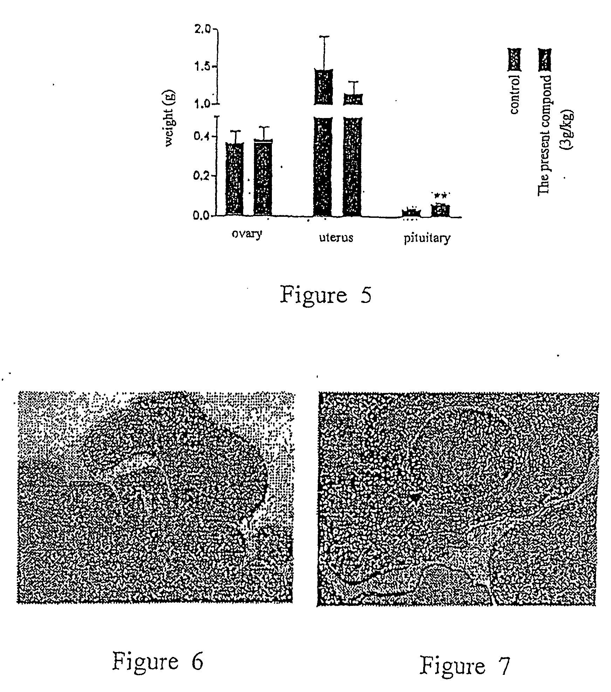

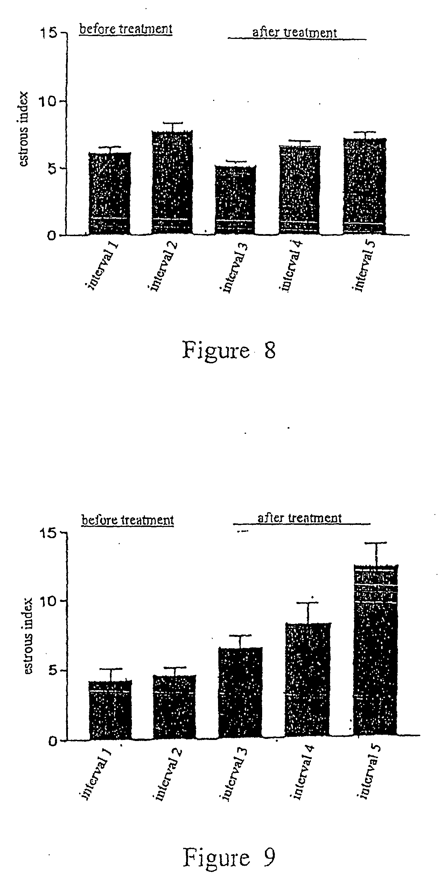

Female SD rats of 18-month age (n > 7) are orally administrated with the present medicaments at 3g/kg for 8 weeks. Then, they are sacrificed and the desired organs are removed. The result shows that the weights of pituitaries in rats treated with the present medicaments are markedly higher than those of untreated rats (Fig. 5). The enlarged folliculus, indicating development of folliculus, can be observed in the ovary of rats treated with the present medicaments (Fig. 7), while no changes are observed in control (Fig. 6). Histological examination of vaginal smear shows that there are a higher percentage of cells having characteristics of proestms or estrus of the ovulatory cycle in animals treated with the present medicaments than the untreated control animals (Fig. 8 and 9), suggesting that the present medicaments may have a estrogen-like effect on portio vaginalis.

### Example 8

### Effects of the present medicaments on ADP-induced platelet aggregation

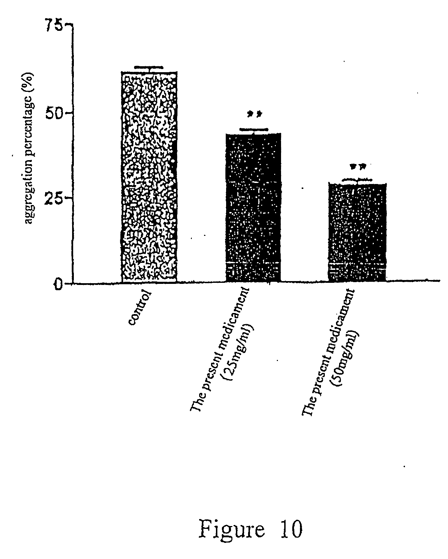

The effects of the present medicaments and components thereof on ADP (15µg/ml)-induced platelet aggregation are screened and tested by collecting blood from a rabbit, treating the blood to obtain the platelet-rich plasma (PRP), and testing the aggregation degree of the platelets in a platelet aggregation meter. Among 16 components tested, 6 components show significant activity against platelet aggregation. The present medicaments have significant activity against platelet aggregation (Fig. 10).

## Claims

1. A medicament for treatment of climacteric syndrome of women, **characterized in that** it is a pharmaceutical formulation comprising the following materials by weight proportion:
| | | | |
|---|---|---|---|
| Radix Ginseng | 3.00-3.52 | Radix Angelicae Sinensis | 6.78-7.96 |
| Radix Astragali seu Hedysari | 11.62-13.64 | Radix Ophiopogonis | 4.31-5.05 |
| Fructus Ligustri Lucidi | 4.31-5.05 | Rhizoma Atractylodis Alba | 4.31-5.05 |
| Radix Paeoniae Alba | 5.56-6.52 | Poria cocos | 6.78-7.96 |
| Cortex Phellodendri | 4.31-5.05 | Rhizoma Ligustici Wallichi | 5.56-6.52 |
| Radix Glycyrrhizae | 4.31-5.05 | Radix Bupleuri | 5.56-6.52 |
| Radix Rehmanniae Praeparata | 11.62-13.64 | Herba Epimedii | 6.78-7.96 |
| Herba Menthae | 2.54-2.98 | Rhizoma Curculiginis | 4.64-5.44 |

2. The medicament for treatment of climacteric syndrome of women according to Claim 1, **characterized in that** it is a pharmaceutical formulation comprising the following materials by weight proportion:
| | | | |
|---|---|---|---|
| Radix Ginseng | 3.00-3.52 | Radix Angelicae Sinensis | 6.78-7.96 |
| Radix Astragali seu Hedysari | 9.78-11.48 | Radix Ophiopogonis | 4.31-5.05 |
| Fructus Ligustri Lucidi | 4.31-5.05 | Rhizoma Atractylodis Alba | 3.40-3.98 |
| Radix Paeoniae Alba | 5.56-6.52 | Poria cocos | 4.94-5.80 |
| Cortex Phellodendri | 4.32-5.06 | Rhizoma Ligustici Wallichi | 3.72-4.36 |
| Radix Glycyrrbizae | 3.40-3.99 | Radix Bupleuri | 5.56-6.52 |
| Radix Rehmanniae Praeparata | 11.62-13.64 | Herba Epimedii | 6.78-7.96 |
| Herba Menthae | 2.54-2.98 | Rhizoma Curculiginis | 4.64-5.44 |
| Rhizoma Anemarrhenge | 3.68-4.32 | Herba Ecliptae | 3.68-4.32 |

3. The medicament for treatment of climacteric syndrome of women according to Claim 1, **characterized in that** it is a pharmaceutical formulation comprising the following materials by weight proportion:
| | | | |
|---|---|---|---|
| Radix Ginseng | 3.26 | Radix Angelicae Sinensis | 7.37 |
| Radix Astragali seu Hedysari | 12.63 | Radix Ophiopogonis | 4.69 |
| Fructus Ligustri Lucidi | 4.69 | Rhizoma Atractylodis Alba | 4.69 |
| Radix Paeoniae Alba | 6.04 | Poria cocos | 7.37 |
| Cortex Phellodendri | 4.69 | Rhizoma Ligustici Wallichi | 6.04 |
| Radix Glycyrrhizae | 4.69 | Radix Bupleuri | 6.04 |
| Radix Rehmanniae Praeparata | 12.63 | Herba Epimedii | 7.37 |
| Herba Menthae | 2.76 | Rhizoma Curculiginis | 5.04 |

4. The medicament for treatment of climacteric syndrome of women according to any one of Claim 1 to 3, **characterized in that** said pharmaceutical formulation is in any one of pharmaceutical acceptable oral dosage forms.

5. The medicament for treatment of climacteric syndrome of women according to Claim 4, **characterized in that** said pharmaceutical formulation is in form of tablets, granules, pills, powders, hard gelatin capsules, soft gelatin capsules, elixirs, troches or oral solutions.

6. The medicament for treatment of climacteric syndrome of women according to Claim 5, **characterized in that** said pharmaceutical formulation is in form of aqueous-honey-bolus.

7. A method for preparing the aqueous-honey-bolus for treatment of climacteric syndrome of women according to Claim 6, **characterized in that**:
Radix Ophiopogonis is premilinarily decocted then formulated; Radix Paeoniae Alba is rinsed with water by a high-speed spray gun; Radix Ginseng is ground separatedly; the others are prepared by traditional processes of preparing raw TCM, followed by crushing;
Except for Radix Ginseng, all materials are mixed together with appropriate amount of wine, vinegar, ginger, salt and the like, followed by decocting in an autoclave;
After decocting, materials are dried at 40-80°C, ground, passed through a sieve of 80 mesh, and mixed with Radix Ginseng powder of the same mesh;
The above mixture comprising afore-mentioned powders, are added water and honey in a proportion to prepare aqueous-honey-bolus.

8. The method for preparing the aqueous-honey-bolus for treatment of climacteric syndrome in women according to Claim 7, in which said drying is at 80°C.
